# EUROPÄISCHE PATENTSCHRIFT

(11) **EP 0 625 041 B1**
(45) Veröffentlichungstag und Bekanntmachung des Hinweises auf die Patenterteilung: **02.11.1995**
(21) Anmeldenummer: 93917385.2
(22) Anmeldetag: 04.02.1993
(51) Int. Cl.: A61K 7/50, A61K 7/08, A61K 7/06

(54) **WÄSSRIGE REINIGUNGSMITTEL**
AQUEOUS CLEANING PRODUCTS
PRODUIT DE LAVAGE AQUEUX

(30) Priorität: 12.02.1992 DE 4204034
(43) Veröffentlichungstag der Anmeldung: 23.11.1994
(73) Patentinhaber: Henkel Kommanditgesellschaft auf Aktien, 40191 Düsseldorf (DE)
(72) Erfinder: MÜLLER, Reinhard, D-5140 Erkelenz 7 (DE); HOLLENBERG, Detlef, D-4006 Erkrath (DE); SEIDEL, Kurt, D-4000 Düsseldorf 13 (DE); MATZIK, Iduna, D-4020 Mettmann (DE)
(86) Internationale Anmeldenummer: EP9300260
(87) Internationale Veröffentlichungsnummer: WO9315714

(56) Entgegenhaltungen:
- DE-A- 3 725 868
- FR-A- 2 466 273
- US-A- 4 140 759
- DATABASE WPIL Section Ch, Week 8934, Derwent Publications Ltd., London, GB; Class A96, AN 89-244468
- DATABASE WPIL Section Ch, Week 9021, Derwent Publications Ltd., London, GB;
- Class D13, AN 90-160455

## Beschreibung

### Gebiet der Erfindung

Die Erfindung betrifft wäßrige Reinigungsmittel, insbesondere Mittel zum Waschen oder Spülen von Haaren, die aufgrund ihrer speziellen Zusammensetzung besonders hautfreundlich sind und sich darüber hinaus durch ihre pflegende Wirkung auszeichnen.

### Stand der Technik

Um eine reinigende Wirkung zu erzielen, enthalten wäßrige Reinigungsmittel üblicherweise oberflächenaktive Verbindungen, wodurch in der Regel die Hautbelastung des Mittels erhöht wird. Dies gilt besonders für die wichtige Klasse der Aniontenside.

Diese erhöhte Hautbelastung sollte im Bereich der Körperreinigungsmittel vermieden werden. Insbesondere bei Produkten, die für eine häufige Anwendung konzipiert sind, zur Reinigung des Intimbereiches dienen oder mit Schleimhäuten in Berührung kommen, ist eine gute Hautverträglichkeit wichtig. Es besteht daher ein ständiger Bedarf an milden wäßrigen Reinigungsmitteln mit gutem Schaumvermögen.

Im Zuge eines sich verändernden Umweltbewußtseins bevorzugt der heutige Verbraucher darüber hinaus in steigendem Maße Produkte, die natürliche Rohstoffe bzw. Inhaltsstoffe enthalten, die nicht oder möglichst wenig chemisch modifiziert sind.

DERWENT Pub. Ltd., London, GB; Class D13, AN 90-160 455 & JP,A,2 102 290 (NIPPON OILS & FATS KK) 13 April 1990 *Zusammenfassung* beschreibt Öl-in-Polyol und Öl-in-Wasser Emulsionen, die durch Erhitzen einer Mischung aus hydriertem Lecithin, Saponin und Polyol und anschließend durch Zugabe von Öl etc. erhalten werden.

Bei der Wahl geeigneter oberflächenaktiver Verbindungen fällt unter diesem Aspekt den Saponinen eine besondere Bedeutung zu. Saponine findet man beispielsweise in der Panamarinde, im Seifenkraut, in der Kastanie, der Rübe, der Sojabohne oder dem Ahorn. Die schaumbildenden Eigenschaften der Saponine wurden schon frühzeitig zu Wasch- und Reinigungszwecken verwendet. In reinem Zustand und in wäßriger Lösung sind Saponine allerdings recht aggressiv zur Haut. Es wurde beobachtet, daß sie - vor allem bei längerer Anwendung - die Haut stark austrocknen und die Entstehung von Hautrissen begünstigen so daß sie sich nicht als solche zur Formulierung von Duschbädern oder Shampoos eignen. Im Gegenteil müßte man aus Gründen des Verbraucherschutzes von der Verwendung von Saponinen in Reinigungsmitteln, die mit der Haut in Kontakt kommen, gerade von Saponinen Abstand nehmen. Denn es ist gut bekannt, daß Saponine neben ihrem Schaumvermögen eine ausgeprägte hämolytische, d. h. den roten Blutfarbstoff auflösende, Wirkung besitzen (vergl. R. Tschesche, G. Wulff, Fortschr. 1973 (30) 564 ff).

Darüber hinaus ist seit langem die pflegende Wirkung von Eigelb und Honig auf Haare bekannt. Die Haare werden dadurch glänzend und geschmeidig. Allerdings belasten diese Komponenten normalerweise die Haare.

Es ist auch bekannt, im Rahmen einer naturheilkundlichen Kombinationsbehandlung mit einem Shampoo aus hellem Bier, Eigelb und weißem Rum gleichzeitig das Haar zu festigen und den Haarboden zu reinigen (vergl. Dr. med. Wolf Ulrich, Gesundes und schönes Haar, Econ Taschenbuch Nr. 20251, Düsseldorf 1977, Seite 129).

Von besonderem Interesse auf dem Gebiet der wäßrigen Reinigungsmittel sind Mittel zum Waschen oder Spülen von Haaren, z. B. Shampoos und abspülbare Haarnachbehandlungsmittel.

Es bestand daher die Aufgabe, Reinigungsmittel auf Basis natürlicher Rohstoffe zu finden, die sich durch gutes Schaumvermögen bei gleichzeitig geringer Hautbelastung auszeichnet.

Es wurde nun überraschend gefunden, daß durch die Abmischung von Saponinen mit Phospholipiden und/oder Oligopeptiden sowie Mono- und/oder Oligosacchariden die reinigende Wirkung der Saponine erhalten bleibt und ihre Hautverträglichkeit darüber hinaus stark verbessert wird.

Gegenstand der Erfindung sind daher wäßrige Reinigungsmittel enthaltend
(A) 0,5 bis 35 Gew.-% eines oder mehrerer Saponine,
(B) 0,1 bis 35 Gew.-% ein oder mehrere Lecithine und/oder Oligopeptide,
(C) 0,1 bis 20 Gew.-% eines oder mehrerer Mono- und/oder Oligosaccharide,
(D) 10 bis 95 Gew.-% Wasser.

Die erfindungsgemäßen Reinigungsmittel bewirken in abspülbaren Formulierungen eine gründliche Reinigung der Haare. Gleichzeitig werden Haare und Kopfhaut gepflegt.

Unter **Saponinen (A)** ist eine Gruppe von im allgemeinen pflanzlichen Glykosiden, die in Wasser kolloidale, seifenartige Lösungen bilden, zu verstehen (vergl. z. B. D.-A. Neumüller, Römpps Chemie-Lexikon, Stuttgart 1975, Seite 3062). Zu den wenigen tierischen Saponinen gehören die Holothurine der Seegurken. Die Saponine werden nach der Art ihrer Aglykone, der Sapogenine, in Triterpenoid- und Steorid-Saponine unterteilt. Die wichtigsten Aglykone der Triterpen-Saponine leiten sich von den Amyrinen und dem in der Haut von Lupinesamen und einigen Pflanzengummen vorkommenden Lupeol ab. Besonders reich an Triterpen-Saponinen ist die Panamarinde, relativ reich auch die Rostkastanie, das Efeu und die Süßholzwurzel. Wichtige Steorid-Saponine sind z. B. die im Fingerhut vorkommenen Saponine Digitonin, Ditonin und Tigonin. Der Zuckeranteil der Sapogenine kann aus Glukose-, Gallaktose-, Pentose- oder Methylpentose-Einheiten bestehen.

Beispiele für besonders geeignete Saponine sind Yucca- und Quillayasaponin, Wurzelsaponine wie die Saponine aus iranischen und levantinischen Seifenwurzeln. In einer bevorzugten Ausführungsform der Erfindung beträgt der Anteil der Saponine (A) 2 bis 25 Gew.-%.

Als **Komponente (B)** der erfindungsgemäßen wäßrigen Reinigungsmittel werden Lecithine und/oder Oligopeptide eingesetzt. Verbindungen (B1) sind Lecithine, wie Ei-Lecithin oder Soja-Lecithin.

Aus praktischen Gründen kann es vorteilhaft sein, **Eigelb** als natürliche und leicht zugängliche Angebotsform für Lecithin einzusetzen. Unter Eigelb ist dabei die in den Eiern von Vogel- und Reptilienarten innerhalb des Eiweißmantels befindliche gelbe Keimkugel zu verstehen. Das Eigelb kann sowohl in flüssiger als auch in pulverisierter Form eingesetzt werden.

Die Herkunft des Eigelbs unterliegt an sich keinen besonderen Beschränkungen. Zur Gewinnung des Eigelbs gut geeignet sind insbesondere die Eier der unterschiedlichsten wildlebenden oder domestizierten Vogelarten, als da z.B. sind: Hühner, Gänse, Enten, Puten, Wachteln, Tauben, Eulen, Kolibris, Möwen, Kiebitze, Papageien, Kuckucke, Strauße oder Pinguine. Es ist jedoch auch möglich, Eigelb zu verwenden, das aus den Eiern von Reptilienarten wie Schildkröten, Krokodilen, Brückenechsen und Schuppen-Kriechtieren stammt. Aufgrund der eingeschränkten Zugänglichkeit ist jedoch das Eigelb wildlebender Arten von untergeordneter Bedeutung. Besonders bevorzugt im Sinne der vorliegenden Erfindung ist jedoch Eigelb, das aus den Eiern von Hausgeflügel und insbesondere den Eiern mitteleuropäischer und/oder nordamerikanischer Haushühner zugänglich ist. Eine spezielle Variante der letztgenannten Eier als Quelle für Eigelb stellen dabei die sogenannten Kräutereier dar, bei denen den Hühnern Mischfutter mit einem Gehalt an etherischen Ölen verabreicht wird (vergl. DE-OS 37 27 735). Der Einsatz von Eigelb aus Kräutereiern empfiehlt sich daher vor allem in denjenigen Fällen, wo das erfindungsgemäße Reinigungsmittel in Form eines Kräutershampoos angeboten werden soll.

Unter **Oligopeptiden (B2)** sind Peptide zu verstehen, die sich aus mehreren Aminosäure-Einheiten zusammensetzen und Molgewichte im Bereich von 200 bis 20.000 aufweisen. Oligopeptide sind durch den Abbau von Polypeptiden (Proteinen) auf hydrolytischem (sauer oder alkalisch) oder enzymatischem Wege oder durch Synthese zugänglich. Besonders bevorzugte Oligopeptide sind wasserlösliche Proteinhydrolysate.

Die Komponente (B) kann in den erfindungsgemäßen Mitteln entweder allein durch Verbindungen des Typs (B1), oder allein durch Verbindungen des Typs (B2) realisiert werden. Sie kann aber auch in Form von Gemischen von (B1) und (B2) eingesetzt werden; besonders gut eignet sich in dieser Hinsicht Eigelb.

In einer bevorzugten Ausführungsform der Erfindung beträgt der Anteil der Komponente (B) 0,5 bis 30 Gew.-%.

Als **Komponente (C)** der erfindungsgemäßen wäßrigen Reinigungsmittel dienen Mono- und/oder Oligosaccharide. Unter Monosacchariden sind im Sinne der allgemein üblichen Nomenklatur Kohlenhydrate wie Glucose, Fructose, Mannose, Allose, Altrose, Gulose, Idose, Galaktose, Talose, Fucose, Erythrose, Rhamnose zu verstehen. Oligosacchariden sind Verbindungen aus zwei bis zehn Monosaccharid-Einheiten. Eine im Sinne der Erfindung besonders geeignete Gruppe von Oligosacchariden stellen die Disaccharide dar. Als Disaccharide eignen sich z.B. Saccharose, Lactose, Maltose, Cellobiose.

Aus praktischen Gründen kann es vorteilhaft sein, Honig als natürliche und leicht zugängliche Angebotsform für die Komponente (C) einzusetzen. Unter Honig ist dabei im Sinne der üblichen Konvention der von Bienen aus dem zuckerhaltigen Saft von Blüten oder anderen Pflanzenausscheidungen gesammelte und im Saugmagen durch Enzyme umgewandelte, in Waben aufgespeicherte süße Stoff zu verstehen. Der in Waben gesammelte Honig enthält etwa noch bis zu 10 % Saccharose, 22 bis 44 % Glucose, 32 bis 49 % Fructose und 17 bis 21,5 % Wasser (vergleiche O.-A. Neumüller, Römpps Chemie Lexikon, 7. Auflage, Stuttgart 1973, Seite 1507). Da es beim Einsatz von Honig als Komponente (C) im wesentlichen auf dessen Gehalt an Glucose bzw. Fructose ankommt, unterliegt die Art des eingesetzten Honigs an sich keiner besonderen Beschränkung.

In einer bevorzugten Ausführungsform der Erfindung beträgt der Anteil der Komponente (C) 0,5 bis 15 Gew.-%.

Die Effektivität der erfindungsgemäßen Reinigungsmittel läßt sich durch den Zusatz geringer Mengen an **Gallensäuren (E)**, z. B. Cholsäure, Taurocholsäure, Glykocholsäure, Lithocholsäure oder Desoxycholsäure, noch steigern. Die Gallensäuren können dabei in freier Form oder in Form ihrer Salze, insbesondere ihrer Alkalimetallsalze oder Ammoniumsalze, eingesetzt werden.

Die Anwesenheit von Gallensäuren bewirkt darüber hinaus infolge ihrer emulgierenden Wirkung eine Verbesserung der Stabilität des Reinigungsmittels. In der Regel weisen die Salze der Gallensäuren bessere Emulgatoreigenschaften auf als die freien Säuren. Innerhalb der Reihe der Alkalisalze nimmt das Emulgiervermögen vom Lithium zum Natrium und Kalium hin zu. Es ist daher besonders bevorzugt, Kaliumsalze, insbesondere das Kaliumsalz der Cholsäure als Komponente (E) einzusetzen.

Es ist dem Fachmann bekannt, daß die Konsistenz wäßriger Emulsionen durch Zugabe sogenannter Verdickungsmittel gezielt beeinflußt werden kann. Es kann daher gewünscht sein, daß die erfindungsgemäßen Emulsionen 0,1 bis 10 Gew.-%, vorzugsweise 0,5 bis 5 Gew.-% eines Verdickungsmittels enthalten, um eine für die jeweilige Anwendung geeignete Viskosität einzustellen. Es ist dabei wiederum bevorzugt, Verdickungsmittel natürlichen Ursprungs zu verwenden. Solche sind z.B. Polysaccharide, insbesondere Xanthan-Gum, Guar-Gum, Agar-Agar, Alginate und Tylosen, Johannisbrot-Kernmehl und Pektine.

Darüber hinaus eignen sich als Verdickungsmittel Carboxymethylcellulose und Hydroxyethylcellulose, höhermolekulare Polyethylenglykolmono- und - -diester von Fettsäuren, Polyacrylate, Polyvinylalkohol und Polyvinylpyrrolidon.

Als Verdickungsmittel können jedoch auch anorganische Elektrolytsalze eingesetzt werden. Dazu eignen sich alle wasserlöslichen Alkali-, Ammonium- und Erdalkalisalze, z. B. die Fluoride, Chloride, Bromide, Sulfate, Phosphate und Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid. Bevorzugt beträgt die Menge des Elektrolytsalzes 0,1 bis 10 Gew.-%, insbesondere 0,2 bis 5 Gew.%.

Die hautschonenden Eigenschaften der erfindungsgemäßen Mittel kommen besonders dann zur Geltung, wenn diese so formuliert werden, daß sie einen pH-Wert in der Nähe des Neutralpunktes der Haut aufweisen. Mittel mit pH-Werten im Bereich von 3,0 - 7,5, insbesondere von 3,5 - 5,8, sind daher bevorzugt.

Die bevorzugt saure Einstellung der erfindungsgemäßen Mittel wirkt sich an sich bereits positiv auf deren Haltbarkeit aus. Es kann jedoch vorgesehen sein, zusätzliche Konservierungsmittel zu verwenden. Dazu eignen sich beispielsweise Ameisensäure, Propionsäure, Salicylsäure, Benzoesäure, Sorbinsäure, Zimtsäure sowie deren Ester und Salze, desweiteren Menthol, Thymol, Eugenol und Lemon Grass Extrakt sowie die in der Anlage zur Kosmetikverordnung angeführten Substanzen. Die Konservierungsmittel werden in den Haarbehandlungsemulsionen üblicherweise in Mengen von 0,1 bis 2 Gew.-% eingesetzt. Die Verwendung von Benzoesäure als Konservierungsmittel ist dabei besonders bevorzugt.

Weiterhin können die erfindungsgemäßen Reinigungsmittel zusätzlich 0,5-20 Gew.-%, insbesondere 1-10 Gew.-%, an ampholytischen und/oder zwitterionischen Tensiden enthalten, die für ihre hautfreundliche Wirkung bekannt sind.

Unter **ampholytischen Tensiden** werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinat, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Im Hinblick auf Produkte, die möglichst nur natürliche Inhaltsstoffe aufweisen sollen, sind im Sinne der Erfindung jedoch solche Mittel bevorzugt, die als Tenside lediglich Saponine enthalten und die dementsprechend frei von synthetischen Tensiden sind.

Die erfindungsgemäßen Mittel können in einer Vielzahl von reinigenden Konsumentenprodukten wie Haarshampoos, Schaumbädern, Duschbädern und flüssigen Seifen Verwendung finden. Insbesondere eignen sie sich für milde Haarshampoos auf Basis natürlicher Rohstoffe.

Die erfindungsgemäßen Reinigsmittel können aber auch zusätzliche Komponenten und Hilfsstoffe enthalten, wie sie aus dem Stand der Technik bekannt sind. Die wichtigsten sind:
**Tenside/Emulgatoren**, z.B. anionaktive Tenside mit Carboxylat-, Sulfonat-, Sulfat- oder Phosphatgruppen wie Seifen, Alkyl- und Arylethersulfate, Fettamine, quartäre Ammonium- und Pyridiniumverbindungen, nichtionische Emulgatoren wie Ethylenoxidaddukte an Alkohole, Carbonsäuren, Partialglyceride und Sorbitanester (vergl. z.B. W. Umbach [Hrsg.], "Kosmetik - Entwicklung, Herstellung und Anwendung kosmetischer Mittel", S.86-87, Stuttgart 1988).
**Ölkomponenten**, z.B. Substanzen wie Paraffinöl, Pflanzenöle, Fettsäureester, Squalan und 2-Octyldodecanol; als Fette und Wachse beispielsweise Walrat, Bienenwachs, Montanwachs, Paraffin und Cetyl-stearylalkohol.
**Lösungsvermittler**, z.B. niedrige ein- oder mehrwertige Alkohole wie Ethanol, Isopropanol, Ethylenglykol, Propylenglykol, Glycerin, 1,3-Butylenglykol und Diethylenglykol.
**Biogenen Wirkstoffe** wie Pflanzenextrakte, Eiweißabbauprodukte und Vitaminkomplexe.
**Feuchthaltemittel**, z.B. Glycerin, Polyglycerine, Sorbit, 1,2-Propandiol, 1,2,3-Butan-triol, Polyethylenglykole, Glucose, Mannit, Xylit, Pyrollidon-Carbonsäure-Salze (PCA), Aminosäuren, Milchsäure.
**Perlglanzmittel** wie Glykoldistearinsäureester, Ethylenglykoldistearat oder Fettsäuremonoglykolester.
**Duftstoffe**, z.B. natürliche Riechstoffe, die durch Destillation, Extraktion oder Pressung aus Pflanzen gewonnen werden sowie synthetisch hergestellte Riechstoffe (vergl. z.B. H.Aebi, E.Baumgartner, H.P.Fiedler, G.Ohloff, "Kosmetika, Riechstoffe und Lebensmittelzusatzstoffe", Stuttgart 1978).
**Antioxidantien**, z.B. Tocopherole, Lecithin, Guajakol, Butylkresol, 4-Methyl-2,6-di-tert.-butyl-phenol (BHT), 4-Methoxy-2(3)tert.-butylphenol (BHA)
**Farbstoffe**, wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968). Die Farbstoffe werden üblicherweise in Konzentrationen von 0,001 bis 0,1 Gew.-%, bezogen auf die gesamte Mischung, eingesetzt.
**pH-Regulatoren:** Weitere Komponenten der erfindungsgemäßen Mittel sind gewünschtenfalls Substanzen, die der Einstellung des pH-Wertes der Mittel dienen.

Die Gesamtmenge der Hilfsstoffe beträgt 0 - 20 Gew.-%, vorzugsweise 0 - 10 Gew.-%.

Zur Herstellung der erfindungsgemäßen Reinigungsmittel werden die Komponenten (A) bis (D) bzw. (A) bis (E) bei 20 bis 40 °C zusammengerührt, wobei in der Regel milchig aussehende Emulsionen erhalten werden. Gewünschtenfalls können anschließend weitere Hilfsstoffe eingearbeitet werden.

Die folgenden Beispiele dienen der Erläuterung der Erfindung und sind nicht einschränkend zu verstehen.

### Beispiele

### 1. Allgemeines

### 1.1. Abkürzungen

In der Kopfzeile der Tabelle 1 sind die erfindungsgemäßen Beispiele mit **B1** bis **B6**, die Vergleiche mit **V1** und **V2** kenntlich gemacht.

### 1.2. Verwendete Substanzen

**Saponin:** Quillaia 200 Extract Powder (Fa. Nor-food / Dänemark)
**Eigelb:** Eigelb flüssig (Fa. E. Klein)
**Honig:** Bienenhonig (Fa. A. Wolf)
**Cholsäure:** Cholsäure,Na-Salz (Fa. Fluka)
**Taurocholsäure:** Taurocholsäure,Na-Salz (Fa. Fluka)
**Guar Gum:** Cosmedia Guar (Fa. Henkel / Düsseldorf)
**Xanthan Gum:** Keltrol F (Fa. Lehmann & Voss / Hamburg)
**Stärke:** Stärke (Fa. Fluka)
**Citruspektin:** Citruspektin (Fa. Fluka)
**Apfelpektin:** Apfelpektin (Type NSS / Fa. Herbstreit KG)
**Texapon N25:** Wäßrige Lösung von Natriumlaurylethersulfat; Aktivsubstanzgehalt: 28 Gew.-% ("Texapon^{(R)} N25"; Fa. Henkel/Düsseldorf)
**Dehyton K:** Wäßrige Lösung eines Fettsäureamid-Derivats mit Betainstruktur der Formel R-COHN-(CH₂)₃-N⁺(CH₃)₂-CH₂-COO⁻; CTFA-Bezeichnung: Cocamidopropyl Betaine; Aktivsubstanzgehalt: 30 Gew.-%; NaCl-Gehalt: 5 Gew.-% (Fa. Henkel/Düsseldorf)

### 2. Ausführungsbeispiele

Es wurden Shampoos gemäß der in der Tabelle 1 angegebenen Zusammensetzung durch gemeinsames Zusammenrühren aller Komponenten bei 40 °C hergestellt.

Nach dem Abkühlen auf Raumtemperatur (20 °C) wurde die Viskosität der milchig aussehenden Emulsionen mit einem Brookfield-Viskosimeter mit einer Tellerspindel gemessen. Die gefundenen Werte sind ebenfalls der Tabelle 1 zu entnehmen.

Die Shampoos der erfindungsgemäßen Beispiele B1 bis B6 sowie der Vergleichsbeispiele V1 und V2 wurden in einer Menge von 5 g auf das nasse Kopfhaar einer Gruppe von 12 Probanden appliziert und nach einer Einwirkzeit von 5 Minuten mit Wasser abgespült. Dabei stellte sich heraus, daß bei gleicher reinigender Wirkung bei V1 und V2 durchweg eine starke Reizung der Kopfhaut auftrat, nicht jedoch bei den Beispielen B1 bis B6. Bei mehrfachem Shampoonieren verursachten die Shampoos gemäß V1 und V2 eine zunehmende Austrocknung der Haut; bei den Shampoos gemäß B1 bis B6 unterblieb dieser von den Probanden als unangenehm empfundene Effekt. Darüber hinaus zeichneten sich die Haare bei der Behandlung mit den Shampoos B1 bis B6 durch einen schönen Glanz und gute Frisierbarkeit sowie einen verbesserten Frisurenhalt aus.

Bei den Beispielen B5 und B6, die gegenüber den Beispielen B1 bis B4 durch einen zusätzlichen Gehalt an einer Gallensäure gekennzeichnet sind, war eine merklich verbesserte Stabilität der Mischung festzustellen. Diese äußerte sich in einer deutlich verbesserten Lagerstabilität.

**Tabelle 1a)**

| | B1 | B2 | B3 | B4 | B5 | B6 | V1 | V2 |
|---|---|---|---|---|---|---|---|---|
| Saponin | 3,0 | 6,0 | 15,0 | 25,0 | 10,0 | 15,0 | 3,0 | 15,0 |
| Eigelb | 15,0 | 2,0 | 30,0 | 25,0 | 8,0 | 25,0 | - | - |
| Honig | 2,0 | 1,0 | 10,0 | 15,0 | 5,0 | 10,0 | - | - |
| Cholsäure | - | - | - | - | - | 0,2 | - | - |
| Taurocholsäure | - | - | - | - | 1,0 | - | - | - |
| Guar Gum | - | - | - | 0,5 | - | - | - | - |
| Xanthan Gum | 1,0 | - | - | - | - | - | - | - |
| Stärke | - | 2,5 | - | - | - | - | - | - |
| Citruspektin | - | 2,5 | 0,5 | - | 0,5 | - | - | - |
| Apfelpektin | - | - | - | 1,0 | - | - | - | - |
| Texapon N25 | - | 5,0 | - | - | - | - | - | - |
| Dehyton K | - | 5,0 | - | - | - | - | - | - |
| NaCl | - | 1,0 | - | - | - | - | - | - |
| Benzoesäure | 0,5 | 0,5 | 0,5 | 0,5 | 0,5 | 0,4 | 0,5 | 0,5 |
| Viskosität [Pas]^{b)} | 6,0 | 8,0 | 8,5 | 9,8 | 7,5 | - ^{c)} | - ^{c)} | -^{c)} |

| | | | | | | | | |
|---|---|---|---|---|---|---|---|---|
| ^{a)} Angaben in Gew.-% Aktivsubstanz; in jeder Spalte entspricht die Differenz zu 100 % dem Anteil an Wasser. | | | | | | | | |
| b) Brookfield-Viskosimeter, Spindel 4, bei 20 Upm und 20 °C gemessen. | | | | | | | | |
| c) Nicht bestimmt. | | | | | | | | |

## Patentansprüche

1. Wäßrige Reinigungsmittel enthaltend
(A) 0,5 bis 35 Gew.-% eines oder mehrerer Saponine,
(B) 0,1 bis 35 Gew.-% ein oder mehrere Lecithine und/oder Oligopeptide
(C) 0,1 bis 20 Gew.-% eines oder mehrerer Mono- und/oder Oligosaccharide,
(D) 10 bis 95 Gew.-% Wasser.

2. Mittel nach Anspruch 1, worin die Komponente (B) ein Ei-Lecithin, Soja-Lecithin und/oder ein Kephalin ist.

3. Mittel nach Anspruch 1, worin die Komponente (B) ein Oligopeptid ist.

4. Mittel nach Anspruch 1, worin die Komponente (B) ein wasserlösliches Proteinhydrolysat ist.

5. Mittel nach Anspruch 1, worin die Komponente (B) eine Mischung aus einem Lecithin und einem Oligopeptid ist.

6. Mittel nach Anspruch 1, worin die Komponente (B) Eigelb ist.

7. Mittel nach Anspruch 1, worin die Komponente (C) Honig ist.

8. Mittel nach einem der Ansprüche 1 bis 7, das frei von synthetischen Tensiden ist.

9. Mittel nach Anspruch 1, das zusätzlich 0,1 bis 10 Gew.-% einer oder mehrer Gallensäuren (E) in freier Form oder in Form ihrer Alkalimetallsalze enthält.

10. Verfahren zur Behandlung von Haaren, wobei ein Mittel nach einem der Ansprüche 1 bis 9 auf das Haar aufgebracht und nach einer Einwirkzeit mit Wasser abgespült wird.

## Claims

1. An aqueous cleaning formulation containing
(A) 0.5 to 35% by weight of one or more saponins,
(B) 0.1 to 35% by weight of one or more lecithins and/or oligopeptides,
(C) 0.1 to 20% by weight of one or more mono- and/or oligosaccharides,
(D) 10 to 95% by weight of water.

2. A formulation as claimed in claim 1, in which component (B) is an egg lecithin, soya lecithin and/or cephalin.

3. A formulation as claimed in claim 1, in which component (B) is an oligopeptide.

4. A formulation as claimed in claim 1, in which component (B) is a water-soluble protein hydrolyzate.

5. A formulation as claimed in claim 1, in which component (B) is a mixture of a lecithin and an oligopeptide.

6. A formulation as claimed in claim 1, in which component (B) is egg yolk.

7. A formulation as claimed in claim 1, in which component (C) is honey.

8. A formulation as claimed in any of claims 1 to 7 which is free from synthetic surfactants.

9. A formulation as claimed in claim 1 which additionally contains 0.1 to 10% by weight of one or more bile acids (E) in free from or in the form of their alkali metal salts.

10. A process for the treatment of hair in which a formulation as claimed in any of claims 1 to 9 is applied to the hair and, after a contact time, is rinsed off with water.

## Revendications

1. Produit de lavage aqueux contenant
(A) 0,5 à 35 % en poids d'une ou de plusieurs saponines,
(B) 0,1 à 35 % en poids d'une ou de plusieurs lécithines et/ou d'un ou plusieurs oligopeptides,
(C) 0,1 à 20 % en poids d'un ou de plusieurs mono- et/ou oligosaccharides,
(D) 10 à 95 % en poids d'eau.

2. Produit selon la revendication 1, dans lequel le composant (B) est une lécithine d'oeuf, une lécithine de soja et/ou une céphaline.

3. Produit selon la revendication 1, dans lequel le composant (B) est un oligopeptide.

4. Produit selon la revendication 1, dans lequel le composant (B) est un hydrolysat de protéine hydrosoluble.

5. Produit selon la revendication 1, dans lequel le composant (B) est un mélange d'une lécithine et d'un oligopeptide.

6. Produit selon la revendication 1, dans lequel le composant (B) est du jaune d'oeuf.

7. Produit selon la revendicatin 1, dans lequel le composant (C) est du miel.

8. Produit selon la revendication 1 à 7, qui est dépourvu de tensio-actifs de synthèse.

9. Produit selon la revendication 1 contenant en outre 0,1 à 10 % en poids d'un ou de plusieurs acides biliaires (E) sous forme libre ou sous la forme de leurs sels de métaux alcalins.

10. Procédé de traitement des cheveux, un produit selon les revendications 1 à 9 étant appliqué sur les cheveux et rincé à l'eau après un temps de pose.
